(19) Europäisches Patentamt

European Patent Office

Office européen des brevets

(11) **EP 0 503 996 B1**

(12) **EUROPEAN PATENT SPECIFICATION**

(45) Date of publication and mention
of the grant of the patent:
**11.09.1996 Bulletin 1996/37**

(51) Int Cl.$^6$: **G05D 11/03**, G01N 33/00,
B01F 3/02

(21) Application number: **92400525.9**

(22) Date of filing: **28.02.1992**

(54) **Critical orifice dilution system and method**

Methode zur Gasverdünnung mittels Düsen zur Erzeugung einer kritischen Strömung und Vorrichtung dafür

Méthode et dispositif de dilution de gaz à l'aide d'un orifice d'écoulement critique

(84) Designated Contracting States:
**CH DE FR GB IT LI SE**

(30) Priority: **01.03.1991 US 662847**

(43) Date of publication of application:
**16.09.1992 Bulletin 1992/38**

(73) Proprietor: **L'AIR LIQUIDE, SOCIETE ANONYME POUR
L'ETUDE ET L'EXPLOITATION DES PROCEDES
GEORGES CLAUDE
F-75321 Paris Cédex 07 (FR)**

(72) Inventors:
• **McAndrew, James J. F.
F-75007 Paris (FR)**
• **Brandt, Michael D.
F-75007 Paris (FR)**

(74) Representative: **Vesin, Jacques et al
L'Air Liquide
Service Propriété Industrielle,
75, quai d'Orsay
75007 Paris (FR)**

(56) References cited:
DE-A- 2 123 961          GB-A- 1 411 078
US-A- 3 521 658

• SOVIET INVENTIONS ILLUSTRATED Week B48, 16 January 1980 Derwent Publications Ltd., London, GB; AN L1921B/48 & SU-A-652 445
• SOVIET INVENTIONS ILLUSTRATED Section Ch, Week 8848, 18 January 1989 Derwent Publications Ltd., London, GB; Class J, AN 88-344607/48 & SU-A-1 392 381
• MEASUREMENT TECHNIQUES vol. 24, no. 3, March 1981, NEW YORK, USA pages 244 - 247; K. S. ROMAN'KO ET. AL.: 'Metrological Certification of a dynamic Gas- mixing Installation'

Description

BACKGROUND OF THE INVENTION

Field of the Invention

The present invention relates generally to gas phase calibration standards for the calibration of analytical instruments and more particularly to field preparation of low concentration gas phase calibration standards.

Description of Related Art

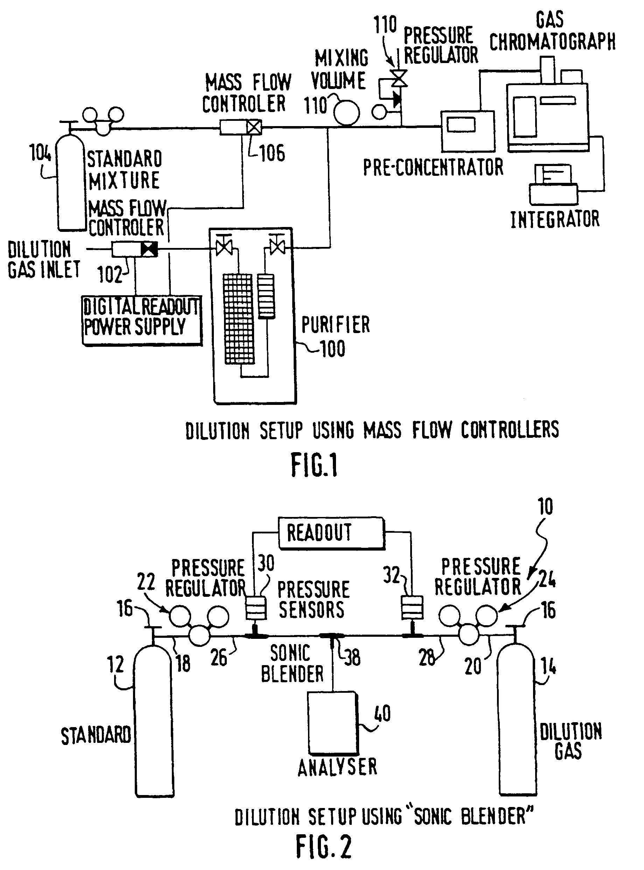

Laboratory or commercial preparation of low concentration standards is known-in the art. For example, a dilution system using mass flow controllers to effect a precise proportion of two gases is shown in Fig. 1. This system comprises a source of pure gas, comprised of a container of gas (not shown) and a purifier 100, whose flow is controlled by a first thermal mass flow control device ("MFC") 102. It further comprises a source of a "standard mixture" 104 (a mixture of pure gas with a known quantity of some impurity) whose flow is controlled by a second MFC 106. The two flows from the MFCs 102 and 106 are combined in a mixing volume 108 such that the ratio of the flows, as regulated by the MFCs 102 and 106, determines the dilution ratio. An excess flow venting device 110 (e.g. a pressure regulator as shown in Fig. 1) provides a means of controlling the flow of the mixed gases to the ultimate destination. This system requires electric power to operate and needs time to reach thermal equilibrium. It is bulky and expensive and not conducive to work in the field. Other commercial systems which operate by combining flows through complicated arrays of capillaries are difficult to manufacture and also very expensive.

In practice, low concentration standards are frequently prepared well in advance of their use and stored in gas cylinders. This pre-mixing to produce a diluted gas of fixed concentration can become a problem when the desired low concentration impurities (typically organic compounds) are suspected of interacting destructively with the internal surfaces of the storage vessel.

For many applications where gas phase calibration standards (consisting of an accurately known concentration of one or more impurities in a balance of pure gas) are required, it is desirable to dilute a standard at high concentration with a pure gas to obtain the desired concentration. Such an approach would avoid many of the problems associated with the storage of premixed standards and provide multiple concentration levels.

Flow of gases through an orifice under "critical" conditions is a relatively well understood phenomenon described in standard texts on fluid dynamics. Several of its features have been employed in a number of different applications.

For example, D.S. Patent No. 4,878,510 to Kasper et al. discloses a method which avoids the formation of droplets by condensation of vapors during the expansion of a highly compressed gas through a critical orifice. Co-pending application Serial No. 107,177 to Wen et al. discloses a method for quantifying the concentration of condensible vapors in a carrier gas by using the minimum pressure drop across a critical orifice to cause droplet formation and then detecting the number of droplets for comparison with the volume of gas which has passed through the critical orifice.

Several applications have employed critical orifices as flow control devices. Soviet Union Patent No. 1,334,109 to Isakov et al. relates to a method to control liquid flow by first vaporizing a liquid, passing the vapor through a critical orifice and then recondensing the liquid. U.S. Patent No. 4,842,827 to Graf et al. describes the use of critical orifices to control the flow of gases to a vacuum system. A high purity "fixed" gas is used to measure the orifice size which is then used to calculate the flow of reactive or toxic gases through the orifice.

Co-pending application Serial Nos. 437,615 and 437,623 to Mettes et al. disclose a process and apparatus to generate low concentration calibration mixtures for testing and/or calibrating analytical instruments such as an atmospheric pressure ionization mass spectrometer. The Mettes inventions employ critical orifices to simultaneously control the flow of multiple gas streams to achieve a known dilution. However, these inventions relate to a system which is large and complicated and is designed for laboratory use. Still further, the system requires multiple mixing steps with more than two gas sources and a moisture generator.

DE-A-2123961 discloses a process and apparatus to mix gases wherein the overall proportions of the gases in the mix are maintained by controlling the pressures of the gases and gas mixture upstream and downstream the mixing chamber.

GB-A-411 078 discloses a gas mixing device to provide a mixture of substantially constant composition, by providing means to control the quantity of each gas before mixing them together.

US-A-3,521,658 discloses a fluid regulator to regulate the pressure of two different fluids. The regulator compensates for changes in the pressure of either fluid to maintain the pressure differential constant.

None of the above prior art relating to the mixing of gases addresses the need of obtaining an excellent mixing of the species, i.e. obtaining a substantially homogeneous gas mixture.

Objects of the Invention

Therefore, it is an object of the present invention to overcome the drawbacks of the prior art.

It is another object of the present invention to provide a portable apparatus capable of producing low concentration gas phase calibration standards.

It is a further object of the present invention to provide an apparatus and method to produce varying low concentrations of gas phase calibration standards.

SUMMARY OF THE INVENTION

According to the present invention, there is provided a gas dilution system with

first (12) and second (14) gas storage means, said first (12) gas storage means containing a pressurized standard gas and said second (14) gas storage means containing a pressurized dilution gas;

first (18) and second (20) conduit means for conducting gas from said first and second gas storage means, respectively;

means (30, 32) for measuring the pressure of gas in at least one of said first and second conduit means;

first (40) and second (42) orifices communicating with said first and second conduit means, respectively, each orifice being calibrated to conduct pressurized gas therethrough at constant velocity to provide respective gas flows in a proportion determined by the sizes of said orifices; and

mixing chamber means (38, 44, 146, 148, 150, 152) for receiving and combining the gases flowing through said first and second orifices,

characterised in that said mixing chamber means comprises an additional mixing chamber in the form of tubing means (146, 148, 150, 152) defining a convoluted path for at least one of the gases, said convoluted path avoiding providing an excellent mixing of the dilution gas and the standard gas while avoiding turbulent flow of said gases.

Preferably, the taking means for defining a convoluted path are such that the flow of gases remains laminar.

**BRIEF DESCRIPTION OF THE DRAWINGS**

In the drawings:

Fig. 1 is a schematic diagram of a dilution system employing a prior art mass flow controller;
Fig. 2 is a schematic diagram of a gas mixing system;
Fig. 3 is a cross-sectional view of the "sonic blender";
Fig. 4 is a graph of the pressure to flow relationship for a typical calibrated orifice;
Fig. 5 is a graph comparing dilution of a standard by a mass flow controller and by the Fig. 2 system;
Fig. 6 is a schematic diagram of a second gas mixing system;
Fig. 7 is a schematic diagram of a third gas mixing system;
Fig. 8 is a cross sectional view of the differential pressure regulator of the third gas mixing system; and
Fig. 9 presents an improved device and method according to the present invention.

**DETAILED DESCRIPTION OF THE INVENTION**

In accordance with the present invention a low concentration gas mixture typically of organic compounds in nitrogen or air (on the order of parts per billion (by volume)), is generated at the time of use by dilution of a parts per million level standard (by volume). The generation of the low concentration standard at the time of use is desirable because it is believed that mixtures at the parts per million level are more stable in gas cylinders than those at the much lower parts per billion level.

Figure 2 depicts a first gas mixing system. In the system of Fig. 2, the system 10 includes two cylinders of gas; a first cylinder 12 of a standard mixture and a second cylinder 14 of a dilution gas. Each cylinder has a standard valve 16 to release the gas from within. Conduits 18 and 20 carry the gas from the cylinders to respective pressure regulators 22 and 24. Preferably, the pressure regulators 22 and 24 include a diaphragm made of metal which will not interact with organic species or contribute impurities to the gas streams.

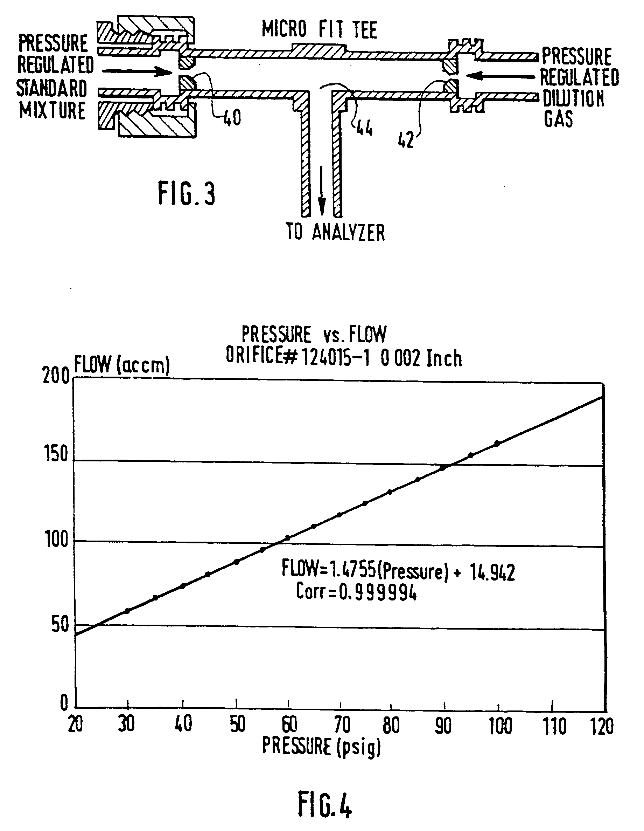

Conduits 26 and 28 carry the gases downstream from the pressure regulators 22 and 24 to high accuracy pressure sensors (or gauges) 30 and 32 which read the pressure of each gas in conduits 26 and 28 and display them to an operator/user. The pressures are adjusted, as necessary, in accordance with the gauge readings to achieve the desired flow rate. The conduits 26 and 28 then carry the pressure regulated gases to a "sonic blender" 38. This mixing apparatus 38 is shown in Fig. 3.

The sonic blender 38 comprises two calibrated orifices 40 and 42 and a 'T'-shaped mixing chamber 44 which directs the final, diluted gas to the device to be calibrated (or to a storage vessel). The exact proportion of the diluted gas is determined by the size of the orifices, as well as the pressure and density of the gases.

The method and apparatus of the present invention relies on the use of "critical" flow through two calibrated orifices (e.g. 40 and 42 in Fig. 3). The velocity of gas through a sufficiently small orifice will eventually reach a maximum value as the differential pressure across the orifice increases. This maximum value is the so-called "critical" flow condition which occurs when the velocity of the gas through the orifice is equal to the local sonic velocity. (In the case of nitrogen, the critical flow condition occurs when the upstream pressure is approximately twice the downstream value pressure.

Gases are made to flow through the two calibrated orifices at sonic velocity (by regulating the upstream pressure) to insure accurate flow control. This system is used to achieve accurate proportional mixing of the two gases, which proportion is ultimately determined by the size of the orifices, the upstream pressure and the density of the gases.

Fig. 4 is a graph showing the linear relationship between flow and pressure for a typical calibrated orifice (.002 inches in diameter in the present case). This linear relationship is described by the following equation:

$$(1) \qquad Flow = 1.4755 \, (P) + 14.942$$

where

$P =$ upstream pressure in psig

or

$$(2) \qquad Flow = 1.4755 \, (P) - 6.75$$

where

$P =$ upstream pressure in psia

In order to approximate this pressure/flow relationship without conducting any measurements, a well known formula may be used (see, e.g. "Fluid Dynamics," J.W. Daily and R.F. Harleman, Addison, Wesley, Ready, 1966).

$$(3) \qquad \text{If } F = Flow \text{ in sccm}$$

$$F = 7.6 \times 10^{8}(A) \, (P)$$

where

$A =$ area of orifice in $m^2$ and
$P =$ upstream pressure in psia

Substituting $A = \pi \, (.001 \text{ in})^2 = 2.03 \times 10^{-9} \, m^2$ yields

$$(4) \qquad F = 1.55(P)$$

As can be seen by comparing the theoretical formula (equation 4) and the formula derived from the direct measurement of the flow (equation 2), a substantial increase in accuracy is achieved by direct measurement versus the theoretic calculated value based on the orifice diameter. The discrepancy between the measured slope (the measured flow value) and the calculated (theoretical) flow value may be attributed to non-ideal orifice shape which causes a difference between its effective and actual size. It may also be due to inaccuracies in orifice size measurement. Similarly, relying on measured results yields a non-zero intercept (F = - 6.75 sccm at P = 0 psia). This is because critical flow does not occur at low pressure (i.e. until the pressure ratio on the opposite sides of the orifice reaches 2:1).

The concentration of a resultant mixture ($C_R$) prepared in accordance with the present system and method can be calculated as follows:

$$(5) \qquad C_R = \frac{F_S C_S + F_D C_D}{F_S + F_D}$$

where F represents gas flow, C represents impurity (organic compound) concentration, the subscript S refers to the standard gas and the subscript D refers to the dilution gas.

If $C_D = 0$, i.e. if the dilution gas is completely free of the impurity of interest then:

$$(6) \qquad C_R = \frac{F_S C_S}{F_S + F_D}$$

The pressure to flow relationship, as defined, can be used to effect a dilution. If a first pressure is selected and achieved for one gas source, the required flow and pressure can be selected for the second gas source to achieve a particular desired concentration. In using a first embodiment of the present invention, as shown in Fig. 2, an operator relies on pressure gauges 30 and 32 associated with the respective gas sources to adjust the gas flow through sonic blender 38 to match the calculated requirements to get the needed concentration.

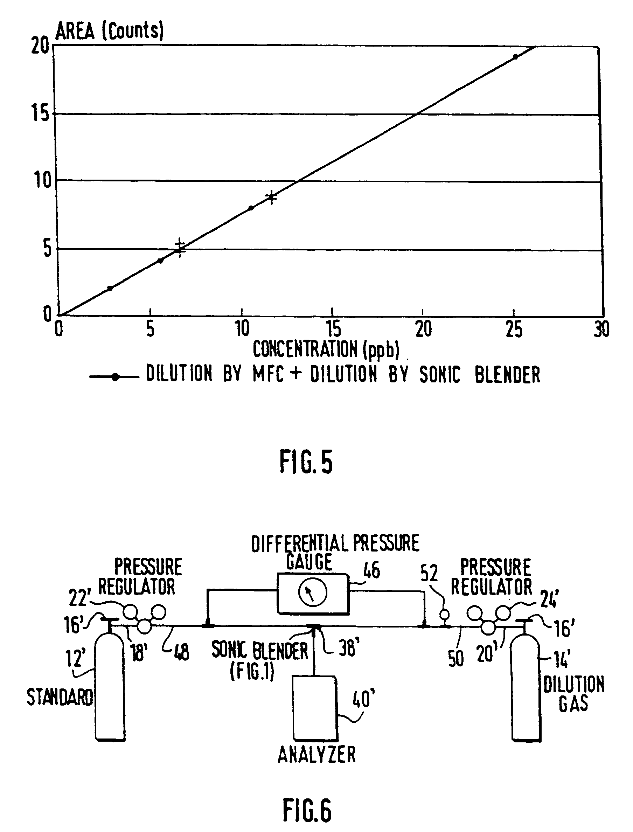

A test was conducted comparing the Fig. 2 system with a high precision mass flow controller based system ("MFC"), as shown in Fig. 1. The analyzer was a gas chromatograph equipped with a flame ionization detector and a pre-concentrator. The results are shown in Fig. 5 as area versus concentration in parts per billion-as generated by both systems. The slope of the line in Fig. 5 is calculated by reading the flows, from the MFCs' digital readouts, which correspond to measured dilutions. Then, the system of Fig. 2 was set to generate flows along the calculated line, and the measured dilutions were plotted. The close agreement between the results obtained through use of the present invention and the results obtained through the MFC reflects the relative accuracy of the present invention. A check of the results obtained against the results calculated through equation 6 verifies the ability to predict the final concentration of the standard based on the pressure/flow relationships.

In the system of Fig. 2, it is possible that errors may be introduced by the pressure readings taken with the pressure sensors 30 and 32 since most, if not all, pressure sensors have some error in their readings. In other words, the use of two pressure measuring devices can result in multiple pressure reading errors. Such errors ultimately effect the accuracy of the dilution.

One way to address this error problem is to use high accuracy pressure sensing devices. Another way to minimize such errors is to rely on a single differential pressure gauge.

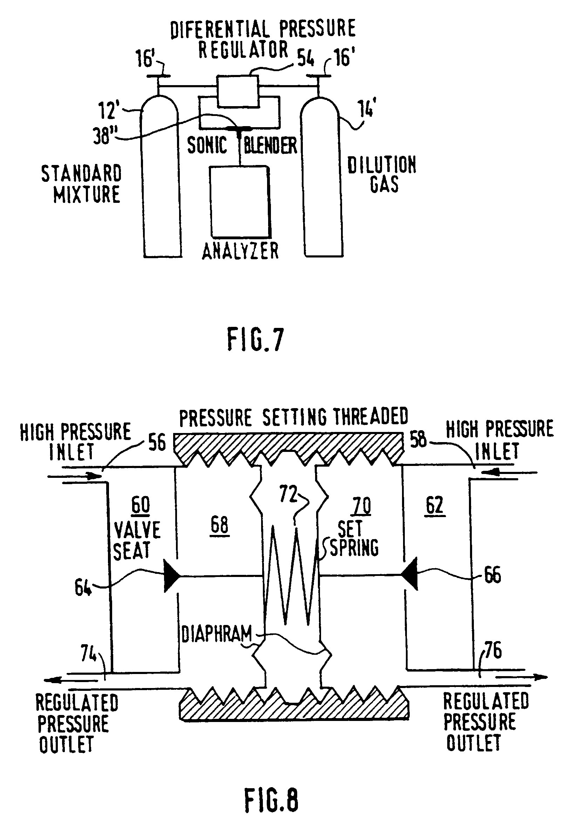

A second gas mixing system, shown in Fig. 6, employs this second solution. Instead of a plurality of pressure sensors, this embodiment relies on a differential pressure gauge 46 which is connected to conduits 48 and 50 on either side of the sonic blender 38′ and an absolute pressure gauge 52 on the dilution or standard gas side (this gauge may be connected to either gas side without impairing the operation of this embodiment).

If it is desired to prepare a particular low concentration standard, the pressure of the dilution gas is first set to the chosen value for the standard side (as indicated on the absolute pressure gauge 52 connected to the dilution gas side). Then, the pressure on the standard side is adjusted until the differential gauge 46 reads zero. This sets the proper pressure on the standard side. Lastly, the desired pressure on the dilution gas side is set according to the absolute pressure gauge 52. (The differential gauge may read off-scale, but as long as its rated pressure is not exceeded, it will not be damaged). In this way, most of the errors associated with two different gauges as in the Fig. 2 system, cancel each other out.

This error cancellation can be illustrated by assuming that the error in the absolute gauge is a constant fraction, $x$, of its reading. Then, the error in $C_R$ will be given by the following equation where P stands for gas pressure and A stands for orifice area:

$$(7) \qquad \Delta C_R = \frac{(1 + x)\, P_S A_S C_S}{(1 + x)\,(P_S A_S + P_D A_D)} - \frac{P_S A_S C_S}{P_S A_S + P_D A_D}$$

$$= 0$$

In this case, the gauge errors cancel exactly. In practice, the assumption of constant percentage error is probably slightly inaccurate. The effect of a non-zero intercept in the pressure-flow relationship which generally occurs in practice (see Fig. 4) is neglected. This error is small and decreases with flow rate. Regardless of these considerations, a considerable reduction in error over a two gauge system is realized.

By way of example, if the absolute pressure gauge 52 is connected to the standard gas side and a pressure of 80 pounds is desired on the dilution gas side with a pressure of 50 pounds on the standard gas side, the pressure of the standard gas first is adjusted until the absolute pressure gauge reads 80 pounds. Then, the pressure of the dilution gas is adjusted until the differential pressure gauge reads 0. This sets the dilution gas pressure to exactly 80 pounds. Lastly, the standard gas pressure is adjusted until the absolute pressure gauge read 50 pounds.

If the absolute pressure gauge is omitted, the system may still deliver a single concentration when the pressures upstream of both orifices are equal. Using a form of the previous flow-concentration relationship, and neglecting the non-zero intercept of the pressure-flow relationship (i.e. assuming $F \simeq PA$):

$$(8) \qquad C_R = \frac{P_S A_S C_S}{P_S A_S + P_D A_D}$$

If $P_S = P_D$, then

$$(9) \qquad C_R = \frac{A_S}{A_S + A_D} \, C_S,$$

independent of pressure.

In practice, the non-zero intercept of the pressure-flow relationship introduces a pressure dependence to the resultant concentration even when the pressures upstream of both orifices are equal. The extent of this pressure dependence will depend on the particular orifices used. In one particular test of a system built in accordance with the present invention, the dilution factor varied from 0.022 at 20 psia to 0.025 at 100 psia with equal pressures upstream. Thus, the omission of the absolute pressure gauge may reduce the accuracy of the system.

In reality, the above considerations are generally of little concern since a regulator, with an absolute pressure gauge is usually used upstream of the critical orifice.

A similar, single concentration approach, is taken in a third system, shown in Figs. 7 and 8. A specialized pressure regulating device 54, which is designed to regulate an equal pressure between the two gases, is employed. As shown in Fig. 8, the device 54 preferably comprises a pair of high pressure inlets 56 and 58 through which the two gases initially flow. A pair of chambers 60 and 62 having valves 64 and 66 therein, receive the gases from the inlets 56 and 58. The action of the valves 64 and 66 is governed by a set spring 72 which provides the appropriate balance to achieve the regulated equal pressure. When the gases are at the appropriate equal pressures (which may be set manually with the set spring 72), the valves 64 and 66 close, in response to the pressure of the gases on the diaphragms 74, to stop the flow of the gases into second chambers 68 and 70. Finally, the regulated pressure gas flows out of the device 54 through outlets 74 and 76 toward the sonic blender 38″. This embodiment thus provides only a single dilution since the regulator does not posses the capacity to vary the relative upstream pressures to the orifices of the sonic blender.

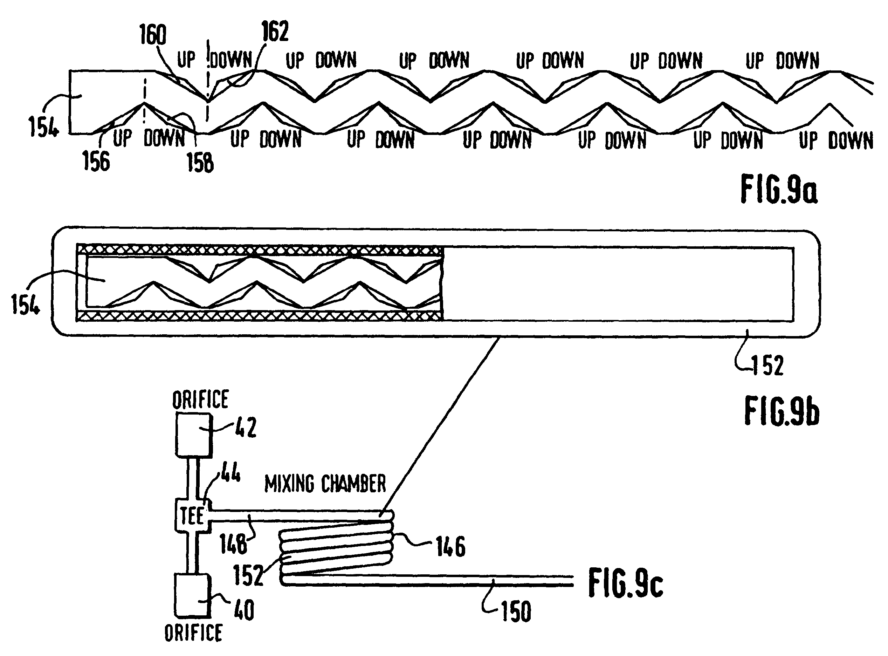

Fig. 9 represents an improved device and method according to the invention, particularly suited for an excellent mixing of the dilution gas and the standard gas, while avoiding turbulent flow of said gases (their flows must remain laminar). This improved mixing is made possible by using an additional 146 mixing chamber wherein at least one of the gases and preferably both gases (or gas mixtures) follow a convoluted path, e.g. in a coiled tubing, in order to increase the distance travelled by an average gas molecule after the mixing point of the two gases (compared to the distance travelled by an average gas molecule without this convoluted path). Preferably, the convoluted path, e.g. the coiled tubing will be provided with means to deflect the flow of at least the standard gas from the wall towards the centerline of the path, e.g. the tubing such as a foil strip with bent cuts as represented on Fig. 9. Those or other means will also preferably breakup the flow stream of both the standard and/or diluent gases into multiple sub-streams.

In order to carry out this mixing in good conditions, it is preferred, according to the invention, to provide a convoluted path sufficiently open to avoid large pressure drops across it, while providing preferably a foil (metal,...) which disrupts the gas flow path so that it is no longer parallel to the tubing walls. Preferably, this foil will contact the tubing walls at multiple points so that the boundary layer is disturbed. To be completely efficient, this convoluted path shall have a sufficient length that the flow is disrupted in the three dimensions and/or that it splits the standard gas flow into sub-streams to increase the speed of the diffusion of the gases.

On Fig. 9a, is shown a foil strip (e.g. in copper, electropolished stainless steel, etc., having a surface treated in a way known by a man skilled in the art to avoid the generation of impurities and/or particles) 154 which is cut to the inside diameter of the tubing being used for the additional mixing chamber 146. Cuts are made on this strip inward toward the centerline of the foil and the edges of the cuts are bent up (like 156, 160,...) or down (158, 162,...), e.g. alternatively.

The foil strip 154 with its cuts is then inserted into a piece of tubing 148, 150, 152 (of adequate material, e.g. as for the foil strip) along the diamater, thus creating a convoluted path in the tube as disclosed above. The tubing 148, 150, 152 with the foil inside (as exemplified on Fig. 9b) is then coiled, the foil strip 154 being present at least on a part of the length of the coiled part 146 of the tubing 148, 150, 152, which represents the additional mixing chamber on Fig. 9c. The end of the tube 148 is then welded to the output end of the Tee 44 while the end of the tube 150 is connected to the analyzer. Preferably, the foil strip contacts the tubing wall not only along the diameter of introduction of said strip in said coil, but also with at least some of the bent cuts along the inside wall of the tubing.

The present invention provides an accurate, portable system for preparing low concentration gas phase calibration standards. This system is used in conjunction with a simple methodology which yields results comparable to other large and more expensive systems.

## Claims

1. A gas dilution system with:

     first (12) and second (14) gas storage means, said first (12) gas storage means containing a pressurized standard gas and said second (14) gas storage means containing a pressurized dilution gas;
first (18) and second (20) conduit means for conducting gas from said first and second gas storage means, respectively;
means (30, 32) for measuring the pressure of gas in at least one of said first and second conduit means;
first (40) and second (42) orifices communicating with said first and second conduit means, respectively, each orifice being calibrated to conduct pressurized gas therethrough at constant velocity to provide respective gas flows in a proportion determined by the sizes of said orifices; and
mixing chamber means (38, 44, 146, 148, 150, 152) for receiving and combining the gases flowing through said first and second orifices,

characterized in that said mixing chamber means comprises an additional mixing chamber in the form of tubing means (14, 148, 150, 152) defining a convoluted path for at least one of the gases, said convoluted path providing an excellent mixing of the dilution gas and the standard gas while avoiding turbulent flow of said gases.

2. A gas dilution system according to claim 1 wherein said tubing means comprises coiled tubing.

3. A gas dilution system according to claim 2 wherein said coiled tubing includes deflection means (154, 156, 158, 160, 162) therein for deflecting the flow of said at least one gas from an inner wall of said tubing toward a centerline of the path of said at least one gas.

4. A gas dilution system according to one of claims 1 to 3, wherein the means for measuring the pressure comprise pressure differential gauge means communicating with said first and second conduit means for comparing the pressure of gas in said first conduit means to the pressure of gas in said second conduit means.

5. A gas dilution system according to claim 4 wherein said means for measuring the pressure comprise sensor means which is disposed in only one of said first and second conduit means and is operable to indicate a desired pressure of the gas flowing in said one conduit means and said pressure differential gauge means is operable to indicate substantially zero differential pressure when the pressure of the gas flowing in the other conduit means is equal, to said desired pressure whereupon said sensor means is operable to indicate a predetermined pressure of the gas flowing in said one conduit means.

6. A gas dilution system according to one of claims 1 to 5 characterized in that it further comprises, as means for defining a convoluted path, means to deflect the flow of at least the standard gas from the walls toward the centerline of the path.

7. A gas dilution system according to one of claims 1 to 5 characterized in that the convoluted path is sufficiently open to avoid large pressure drop across it, and the flow path is no longer parallel to the walls.

8. A gas dilution system according to one of claims 1 to 7, wherein the means for defining a convoluted path comprise a tubing with a foil therein, said foil contacting the tubing walls at multiple points so that the boundary layer is disturbed.

9. A gas dilution system according to one of claims I to 8, wherein the convoluted path has a sufficient length such that the flow of gas is disrupted in the three dimensions and/or that it splits the standard gas flow into substreams to increase the speed of diffusion of the gases.

10. A gas dilution system according to one of claims 1 to 9 wherein the convoluted path comprises a foil strip with cuts therein.

**Patentansprüche**

1. Ein Gasverdünnungssystem mit:

erstem (12) und zweitem (14) Gasspeichermittel, wobei das erste (12) Gasspeichermittel ein komprimiertes Standardgas enthält und das zweite (14) Gasspeichermittel ein komprimiertes Verdünnungsgas enthält;
erstes (18) und zweites (20) Leitungsmittel zum Leiten von Gas vom ersten bzw. zweiten Gasspeichermittel;
Mittel (30, 32) zur Messung des Druckes von Gas in mindestens einem der ersten und zweiten Leitungsmittel;

erste (40) und zweite (42) Öffnungen, welche mit dem ersten bzw. dem zweiten Leitungsmittel in Verbindung stehen, wobei jede Öffnung kalibriert worden ist, um komprimiertes Gas dort mit konstanter Geschwindigkeit durchzuleiten, um jeweilige Gasströme in einem Verhältnis zur Verfügung zu stellen, welches durch die Größe besagter Öffnungen festgelegt ist;

Mischungskammermittel (38, 44, 146, 148, 150, 152) zum Empfangen und Verbinden der Gase, welche durch diese erste und zweite Öffnungen fließen,

dadurch gekennzeichnet, daß das Mischungskammermittel eine zusätzliche Mischungskammer in der Form von Rohrleitungsmitteln (146, 148, 150, 152) enthält, welche einen gewundenen Pfad für mindestens eines der Gase definieren, wobei der gewundene Pfad eine ausgezeichnete Mischung des Verdünnungsgases und des Standardgases bei Vermeidung turbulenter Strömung dieser Gase zur Verfügung stellt.

2. Ein Gasverdünnungssystem nach Anspruch 1, wobei besagtes Rohrleitungsmittel eine gewendelte Rohrleitung enthält.

3. Ein Gasverdünnungssystem nach Anspruch 2, wobei diese gewendelte Rohrleitung Richtungsänderungsmittel (154, 156, 158, 160, 162) darin zum Ablenken der Strömung von diesem mindestens einem Gas von einer inneren Wand dieser Rohrleitung zu einer Mittellinie des Weges von diesem mindestens einem Gas enthält.

4. Ein Gasverdünnungssystem nach einem der Ansprüche 1 bis 3, wobei die Mittel zur Messung des Druckes Differenzialdruckmeßmittel enthalten, welche mit dem ersten und zweiten Leitungsmittel zum Vergleichen des Gasdruckes in dem ersten Leitungsmittel zum Gasdruck im zweiten Leitungsmittel kommunizieren.

5. Ein Gasverdünnungssystem nach Anspruch 4, wobei die Mittel zur Messung des Druckes Sensormittel enthalten, welches nur in einem der besagten ersten und zweiten Leitungsmittel angeordnet und betriebsfähig ist, einen gewünschten Druck des Gases, welches in diesem einen Leitungsmittel strömt, anzuzeigen, und besagtes Differenzialdruckmeßmittel betriebsfähig ist, im wesentlichen Null-Differenzialdruck anzuzeigen, wenn der Druck des Gases, welches in dem anderen Leitungsmittel strömt, gleich zu besagtem gewünschten Druck ist, woraufhin besagtes Sensormittel betriebsfähig ist, einen vorherbestimmten Druck des Gases anzuzeigen, welches in besagtem einen Leitungsmittel strömt.

6. Ein Gasverdünnungssystem nach einem der Ansprüche 1 bis 5, dadurch gekennzeichnet, daß es weiterhin als Mittel zur Definierung eines gewundenen Pfades Mittel zum Ablenken der Strömung von mindestens dem Standardgas von den Wänden zu der Mittellinie des Pfades enthält.

7. Ein Gasverdünnungssystem nach einem der Ansprüche 1 bis 5, dadurch gekennzeichnet, daß der gewundene Pfad hinreichend geöffnet ist, um einen großen Druckabfall über diesen zu vermeiden, und daß der Strömungspfad nicht länger parallel zu den Wänden ist.

8. Ein Gasverdünnungssystem nach einem der Ansprüche 1 bis 7, wobei die Mittel zur Definierung eines gewundenen Pfades eine Rohrleitung mit einer darin enthaltenen Folie enthalten, wobei diese Folie die Rohrleitungswände an mehreren Punkten so berührt, daß die Grenzschicht gestört ist.

9. Ein Gasverdünnungssystem nach einem der Ansprüche 1 bis 8, wobei der gewundene Pfad eine hinreichende Länge hat, so daß die Strömung an Gas in die drei Dimensionen getrennt ist und/oder daß er die Standardgasströmung in Unterströmungen aufteilt, um die Diffusionsgeschwindigkeit der Gase zu steigern.

10. Ein Gasverdünnungssystem nach einem der Ansprüche 1 bis 10, wobei der gewundene Pfad ein Folienband mit Einschnitten darin enthält.

**Revendications**

1. Système de dilution de gaz avec:

un premier (12) et un deuxième (14) moyens de stockage de gaz, ledit premier (12) moyen de stockage de gaz contenant un gaz étalon sous pression et ledit deuxième (14) moyen de stockage de gaz contenant un gaz de dilution sous pression;

un premier (18) et un deuxième (20) moyens de conduit pour acheminer le gaz desdits premier et deuxième moyens de stockage de gaz respectivement;

des moyens (30, 32) pour la mesure de la pression de gaz dans au moins un desdits premier et deuxième moyens de conduit;

un premier (40) et un deuxième (42) orifices communiquant avec lesdits premier et deuxième moyens de conduit, respectivement, chaque orifice étant calibré de manière à y acheminer du gaz sous pression à vitesse constante pour fournir des débits de gaz respectifs dans une proportion déterminée par les tailles desdits orifices; et

un moyen de chambre de mélange (38, 44, 146, 148, 150, 152) pour la réception et la combinaison des gaz s'écoulant à travers lesdits premier et deuxième orifices, caractérisé en ce que ledit moyen de chambre de mélange comprend une chambre de mélange supplémentaire sous la forme de moyens de tubulure (146, 148, 150, 152), définissant un trajet convoluté pour au moins l'un des gaz, ledit trajet convoluté fournissant un excellent mélange du gaz de dilution et du gaz étalon, tout en évitant un écoulement turbulent desdits gaz.

2.  Système de dilution de gaz selon la revendication 1, dans lequel ledit moyen de tubulure comprend une tubulure en forme de spirale.

3.  Système de dilution de gaz selon la revendication 2, dans lequel ladite tubulure en forme de spirale inclut des moyens de déviation (154, 156, 158, 160, 162) en son sein pour dévier le courant de l'un au moins desdits gaz d'une paroi interne de ladite tubulure en direction d'une ligne centrale du trajet de l'un au moins desdits gaz.

4.  Système de dilution de gaz selon l'une quelconque des revendications 1 à 3, dans lequel les moyens de mesure de la pression comprennent un moyen de manomètre différentiel communiquant avec lesdits premier et deuxièmes moyens de conduit, pour comparer la pression de gaz dans ledit premier moyen de conduit à la pression de gaz dans ledit deuxième moyen de conduit.

5.  Système de dilution de gaz selon la revendication 4, dans lequel lesdits moyens de mesure de la pression comprennent un moyen de détection, qui est disposé dans l'un seulement desdits premier et deuxième moyens de conduit, et qui peut être mis en service pour indiquer une pression désirée du gaz s'écoulant dans ledit moyen de conduit, et ledit moyen de manomètre différentiel peut être mis en service pour indiquer une pression différentielle effectivement nulle, quand la pression du gaz s'écoulant dans l'autre moyen de conduit est égale à ladite pression souhaitée, à la suite de quoi ledit moyen de détection peut être mis en service pour indiquer une pression prédéterminée du gaz s'écoulant dans ledit moyen de conduit.

6.  Système de dilution de gaz selon l'une quelconque des revendications 1 à 5, caractérisé en ce qu'il comprend en outre, en tant que moyen de définition d'un trajet convoluté, des moyens pour dévier au moins le courant du gaz étalon des parois en direction de la ligne centrale du trajet.

7.  Système de dilution selon l'une quelconque des revendications 1 à 5, caractérisé en ce que le trajet convoluté est suffisamment ouvert pour éviter une chute de pression trop forte en son sein, et en ce que le trajet d'écoulement n'est plus parallèle aux parois.

8.  Système de dilution de gaz selon l'une quelconque des revendications 1 à 7, dans lequel les moyens de définition d'un trajet convoluté comprennent une tubulure ayant en son sein une feuille, ladite feuille entrant en contact avec les parois de la tubulure en des points multiples, de sorte que la couche limitrophe soit perturbée.

9.  Système de dilution de gaz selon l'une quelconque des revendications 1 à 8, dans lequel le trajet convoluté a une longueur suffisante de sorte que l'écoulement de gaz soit détourné dans les trois dimensions et/ou de sorte qu'il fractionne l'écoulement de gaz étalon en des courants subsidiaires pour augmenter la vitesse de diffusion des gaz.

10. Système de dilution de gaz selon l'une quelconque des revendications 1 à 9, dans lequel le trajet convoluté comprend un ruban de feuille contenant des entailles.

DILUTION SETUP USING MASS FLOW CONTROLLERS

FIG.1

DILUTION SETUP USING "SONIC BLENDER"

FIG.2

PRESSURE
REGULATED
STANDARD
MIXTURE

MICRO FIT TEE

40

44    42

TO ANALYZER

PRESSURE
REGULATED
DILUTION
GAS

## FIG. 3

PRESSURE vs. FLOW
ORIFICE # 124015−1  0 002 Inch

FLOW (accm)

FLOW=1.4755(Pressure) + 14.942
Corr=0.999994

PRESSURE (psig)

## FIG. 4

FIG.5

FIG.6

DIFERENTIAL PRESSURE
REGULATOR   54

16'                            16'

12'

38"

SONIC   BLENDER

STANDARD
MIXTURE

DILUTION
GAS

ANALYZER

**FIG.7**

PRESSURE SETTING THREADED

HIGH PRESSURE   56                          58   HIGH PRESSURE
INLET                                              INLET

72

60                    68        70        62
VALVE                           SET
SEAT                           SPRING

64                                            66

DIAPHRAM

74                                            76

REGULATED                              REGULATED
PRESSURE                                PRESSURE
OUTLET                                  OUTLET

**FIG.8**

FIG.9a

FIG.9b

FIG.9c